# EUROPÄISCHE PATENTANMELDUNG

(11) **EP 4 245 410 A1**
(43) Veröffentlichungstag der Anmeldung: **20.09.2023**
(21) Anmeldenummer: 22163016.3
(22) Anmeldetag: 18.03.2022
(51) Int. Cl.: B01J 31/24, B01J 31/22, C07C 45/50

(54) **VERFAHREN ZUR HYDROFORMYLIERUNG VON OLEFINEN MIT EINEM COBALT-PRÄKATALYSATOR UND EINEM DIPHOSPHIN-LIGANDEN**

(71) Anmelder: Evonik Operations GmbH, 45128 Essen (DE)
(72) Erfinder: ZHANG, Boaxin, 18106 Rostock (DE); BÖRNER, Armin, 18059 Rostock (DE); FRANKE, Robert, 45772 Marl (DE)
(74) Vertreter: Evonik Patent Association

(57) **Zusammenfassung**

Verfahren zur Hydroformylierung von Olefinen mit einem Cobalt-Präkatalysator (bevorzugt [Co(acac)(C4H8O2)4]+[BF4]-]) und einem ethylenverbrückten Diphosphin-Liganden.

## Beschreibung

Die Erfindung betrifft ein Verfahren zur Hydroformylierung von Olefinen mit einem Cobalt-Präkatalysator und einem Diphosphin-Liganden.

In Hood et al., "Highly active cationic cobalt (II) hydroformylation catalysts", Science, Band 367, Nummer 6477, Seiten 542-548 (2020) wird der Einsatz von Cobalt-Katalysatoren in der Hydroformylierung beschrieben. Hierbei wurden die verwendeten Diphosphin-Liganden immer im Verhältnis 1:1 zum Cobalt eingesetzt. Bei Rh wurde der Ligand in einem deutlichen Überschuss (L/Rh = 400 - 1600:1) eingesetzt.

Die technische Aufgabe, welche der vorliegenden Erfindung zugrunde lag, ist die Bereitstellung eines Verfahrens, mit welchen Olefine hydroformyliert werden können. In dem Verfahren soll eine gesteigerte Ausbeute erzielt werden.

Diese Aufgabe wird gelöst durch ein Verfahren nach Anspruch 1.

Verfahren umfassend die Verfahrensschritte:
a) Zugabe eines Olefins;
b) Zugabe eines Cobalt-Präkatalysators;
c) Zugabe eines Liganden (L), der die Struktur (I) aufweist: 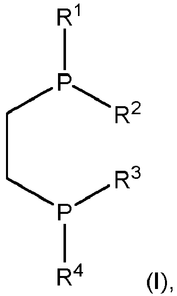
   wobei R¹, R², R³, R⁴ ausgewählt sind aus:

      -H, -(C₁-C₁₂)-Alkyl, -(C₆-C₂₀)-Aryl,
   wobei der Ligand in einem molaren Verhältnis zu Cobalt zugegeben wird von L/Co = <0,95/1;
d) Zuführen von Synthesegas;
e) Erwärmen des Reaktionsgemisches aus a) bis d), wobei das Olefin zu einem Aldehyd umgesetzt wird.

Hierbei können die Verfahrensschritte a) bis d) in beliebiger Reihenfolge erfolgen.

Dass der Ligand in einem molaren Verhältnis zu Cobalt zugegeben wird von L/Co = kleiner 0,95 zu 1 bedeutet, dass der Ligand (L) also im Unterschuss gegenüber dem Metall (Co) zugegeben wird.

In einer Variante des Verfahrens sind R¹, R², R³, R⁴ ausgewählt aus: -(C₁-C₁₂)-Alkyl, -(C₆-C₂₀)-Aryl.

In einer Variante des Verfahrens stehen R¹, R² für -(C₆-C₂₀)-Aryl.

In einer Variante des Verfahrens stehen R¹, R² für -Ph.

In einer Variante des Verfahrens stehen R¹, R³ für -(C₆-C₂₀)-Aryl.

In einer Variante des Verfahrens stehen R¹, R³ für -Ph.

In einer Variante des Verfahrens weist der Ligand die Struktur (1) auf:

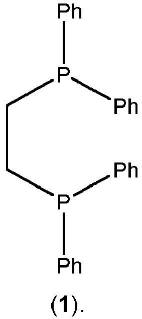

In einer Variante des Verfahrens wird der Ligand in einem molaren Verhältnis zu Cobalt zugegeben von L/Co = <0,90 / 1.

In einer Variante des Verfahrens wird der Ligand in einem molaren Verhältnis zu Cobalt zugegeben von L/Co = <0,80 / 1.

In einer Variante des Verfahrens wird der Ligand in einem molaren Verhältnis zu Cobalt zugegeben von L/Co = <0,70 / 1.

In einer Variante des Verfahrens wird der Ligand in einem molaren Verhältnis zu Cobalt zugegeben von L/Co = <0,60 / 1.

In einer Variante des Verfahrens wird der Ligand in einem molaren Verhältnis zu Cobalt zugegeben von L/Co = <0,50 / 1.

In einer Variante des Verfahrens wird der Ligand in einem molaren Verhältnis zu Cobalt zugegeben von L/Co = <0,40 / 1.

In einer Variante des Verfahrens wird der Ligand in einem molaren Verhältnis zu Cobalt zugegeben von L/Co = <0,30 / 1.

In einer Variante des Verfahrens wird der Ligand in einem molaren Verhältnis zu Cobalt zugegeben von L/Co = <0,20 / 1.

In einer Variante des Verfahrens wird der Cobalt-Präkatalysator als Lösung vorgelegt.

In einer Variante des Verfahrens ist wird das Olefin zu der vorgelegten Cobalt-Präkatalysator-Lösung zugegeben.

In einer Variante des Verfahrens erfolgt das Zuführen von Synthesegas in Verfahrensschritt d) mit einem Druck in dem Bereich von 1 bis 8 MPa (10 bis 80 bar).

In einer Variante des Verfahrens erfolgt das Zuführen von Synthesegas in Verfahrensschritt d) mit einem Druck in dem Bereich von 4 bis 6 MPa (40 bis 60 bar).

In einer Variante des Verfahrens erfolgt das Erwärmen des Reaktionsgemisches in Verfahrensschritt e) auf eine Temperatur in dem Bereich von 80 °C bis 180 °C.

In einer Variante des Verfahrens erfolgt das Erwärmen des Reaktionsgemisches in Verfahrensschritt e) auf eine Temperatur in dem Bereich von 120 °C bis 160 °C.

In einer Variante des Verfahrens handelt es sich bei dem Cobalt-Präkatalysators um [Co(acac)(C₄H₈O₂)₄]⁺[BF₄]⁻(V1).

In einer Variante des Verfahrens ist das Olefin ausgewählt aus:
Ethen, Propen, 1-Buten, cis-2-Buten, trans-2-Buten, Gemisch aus cis- und trans-2-Buten, Raffinat 1, Raffinat 2, Isobuten, 1-Penten, 2-Penten, 2-Methyl-1-buten, 2-Methyl-2-buten, 1-Methylcyclohexen, Tetramethylethen, 1-Octen, 2-Octene, Cycloocten, Di-n-Buten, Di-iso-Buten, Undecene, Dodecene, Triisobuten, Tri-n-Buten.

In einer Variante des Verfahrens ist das Olefin ausgewählt aus:
1-Buten, cis-2-Buten, trans-2-Buten, Isobuten, 1-Penten, 2-Penten, 1-Octen, 2-Octene, Di-n-Buten, Di-iso-Buten, Triisobuten, Tri-n-Buten.

Im Folgenden soll die Erfindung anhand eines Ausführungsbeispiels näher erläutert werden.

### Hydroformylierung von 1-Octen mit [Co(acac)(C₄H₈O₂)](BF₄)

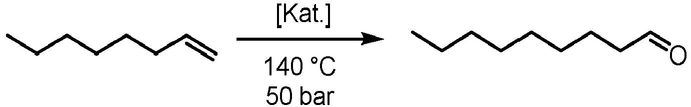

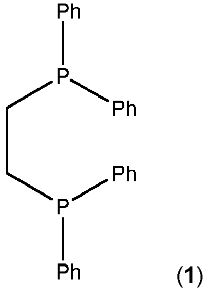

### (1): 1,2-Bis(diphenylphosphino)ethan (DPPE)

### (V1): [Co(acac)(C₄H₈O₂)](BF₄)

Eine Lösung des Co-Präkatalysators (**V1**) und Diphosphins (**1**) in Diglyme (24 mL) wurde in einen 150 mL Premex Autoklaven mit Begasungsrührer eingefüllt und mit 32 bar Synthesegas beaufschlagt. Die Lösung wurde bis auf 160 °C aufgeheizt und der Druck auf 50 bar nachreguliert. Nach 5 Minuten wurde die Temperatur auf 140 °C abgesenkt. Dann wurde das Olefin (4,74 mL) durch eine Druckpipette in die Katalysatorlösung zugefügt und die Reaktionslösung (1 M Olefin) bei 50 bar und 140 °C gerührt. Dann wurde die Reaktionsmischung auf Raumtemperatur abgekühlt und der Druck abgelassen. Die Ausbeuten wurden mittels Gaschromatographie bestimmt.

Reaktionsbedingungen:
[Co]: 2,6 mM, T: 140 °C, p(Synthesegas): 50 bar, t: 1 h, Präkatalysator: **V1**

### Hydroformylierung 1-Octen:

| Ligand | Präkatalysator | P/Co | L/Co | T (°C) | t (h) | Ausbeute (%) |
|---|---|---|---|---|---|---|
| (**1**) | **V1** | 2:1 | 1:1 | 140 | 1 | 35,9 |
| (**1**)* | **V1** | 0,23:1 | 0,115:1 | 140 | 1 | 69,9 |

| | | | | | | |
|---|---|---|---|---|---|---|
| * erfindungsgemäßes Verfahren | | | | | | |

Mit dem erfindungsgemäßen Ligand-Cobalt-Verhältnis (L/Co) konnte die Ausbeute gesteigert werden.

Wie das Ausführungsbeispiel zeigt, wird die Aufgabe durch das erfindungsgemäße Verfahren gelöst.

## Patentansprüche

1. Verfahren umfassend die Verfahrensschritte:
a) Zugabe eines Olefins;
b) Zugabe eines Cobalt-Präkatalysators;
c) Zugabe eines Liganden (L), der die Struktur (**I**) aufweist: 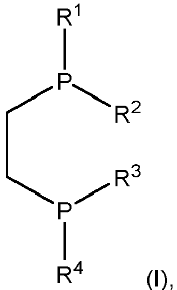
wobei R¹, R², R³, R⁴ ausgewählt sind aus:
-H, -(C₁-C₁₂)-Alkyl, -(C₆-C₂₀)-Aryl,
wobei der Ligand in einem molaren Verhältnis zu Cobalt zugegeben wird von L/Co = <0,95 /1;
d) Zuführen von Synthesegas;
e) Erwärmen des Reaktionsgemisches aus a) bis d), wobei das Olefin zu einem Aldehyd umgesetzt wird.

2. Verfahren nach Anspruch 1,
wobei R¹, R², R³, R⁴ ausgewählt sind aus: -(C₁-C₁₂)-Alkyl, -(C₆-C₂₀)-Aryl.

3. Verfahren nach einem der Ansprüche 1 oder 2,
wobei R¹, R² für -Ph stehen.

4. Verfahren nach einem der Ansprüche 1 bis 3,
wobei R¹, R³ für -Ph stehen.

5. Verfahren nach einem der Ansprüche 1 bis 4,
wobei der Ligand die Struktur (**1**) aufweist:

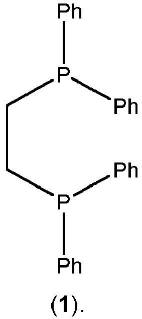

6. Verfahren nach einem der Ansprüche 1 bis 5,
wobei der Ligand in einem molaren Verhältnis zu Cobalt zugegeben wird von L/Co = <0,90 / 1.

7. Verfahren nach einem der Ansprüche 1 bis 6,
wobei der Ligand in einem molaren Verhältnis zu Cobalt zugegeben wird von L/Co = <0,80 / 1.

8. Verfahren nach einem der Ansprüche 1 bis 7,
wobei der Co-Präkatalysator als Lösung vorgelegt wird.

9. Verfahren nach Anspruch 8,
wobei das Olefin zu der vorgelegten Co-Präkatalysator-Lösung zugegeben wird.

10. Verfahren nach einem der Ansprüche 1 bis 9,
wobei das Zuführen von Synthesegas in Verfahrensschritt d) mit einem Druck in dem Bereich von 1 bis 8 MPa (10 bis 80 bar) erfolgt.

11. Verfahren nach einem der Ansprüche 1 bis 10,
wobei das Erwärmen des Reaktionsgemisches in Verfahrensschritt e) auf eine Temperatur in dem Bereich von 80 °C bis 180 °C erfolgt.

12. Verfahren nach einem der Ansprüche 1 bis 11,
wobei es sich bei dem Cobalt-Präkatalysators um [Co(acac)(C₄H₈O₂)₄]⁺[BF₄]-(**V1**) handelt.

13. Verfahren nach einem der Ansprüche 1 bis 12,
wobei das Olefin ausgewählt ist aus:
Ethen, Propen, 1-Buten, cis-2-Buten, trans-2-Buten, Gemisch aus cis- und trans-2-Buten, Raffinat 1, Raffinat 2, Isobuten, 1-Penten, 2-Penten, 2-Methyl-1-buten, 2-Methyl-2-buten, 1-Methylcyclohexen, Tetramethylethen, 1-Octen, 2-Octene, Cycloocten, Di-n-Buten, Di-iso-Buten, Undecene, Dodecene, Triisobuten, Tri-n-Buten.
